# EUROPÄISCHE PATENTSCHRIFT

(11) **EP 3 900 639 B1**
(45) Veröffentlichungstag und Bekanntmachung des Hinweises auf die Patenterteilung: **19.08.2026**
(21) Anmeldenummer: 21401020.9
(22) Anmeldetag: 20.04.2021
(51) Int. Cl.: A61B 1/01, A61B 13/00, A61B 17/24, A61B 1/00, A61B 10/00, A61B 90/30, A61B 90/00, A61B 50/20, A61B 10/02, A61B 1/24

(54) **MEDIZINTECHNISCHE HALTEVORRICHTUNG ZUM HALTEN VON ALLGEMEINMEDIZINISCHEN, CHIRURGISCHEN ODER DIAGNOSTISCHEN EINRICHTUNGEN ODER INSTRUMENTEN SOWIE DEREN VERWENDUNG INSBESONDERE ZUR DATENERFASSUNG**
MEDICAL HOLDING DEVICE FOR HOLDING GENERAL MEDICAL, SURGICAL OR DIAGNOSTIC DEVICES OR INSTRUMENTS AND THEIR USE, IN PARTICULAR FOR DATA ACQUISITION
DISPOSITIF DE MAINTIEN DE LA TECHNIQUE MÉDICALE PERMETTANT DE MAINTENIR DES DISPOSITIFS OU DES INSTRUMENTS MÉDICAUX GÉNÉRAUX, CHIRURGICAUX OU DIAGNOSTIQUES, AINSI QUE SON UTILISATION, EN PARTICULIER POUR L'ACQUISITION DE DONNÉES

(30) Priorität: 21.04.2020 DE 202020102208 U
(43) Veröffentlichungstag der Anmeldung: 27.10.2021
(73) Patentinhaber: Merse, Stefanie, 45147 Essen (DE)
(72) Erfinder: Merse, Stefanie, 45147 Essen (DE)
(74) Vertreter: Tanner, Lukas

(56) Entgegenhaltungen:
- EP-A1- 1 380 829
- WO-A1-2005/084555
- WO-A2-2013/012256
- CN-A- 112 315 505
- US-A- 4 892 831

## Beschreibung

### TECHNISCHES GEBIET

Die vorliegende Erfindung betrifft eine medizintechnische Haltevorrichtung zum Halten von allgemeinmedizinischen, chirurgischen oder diagnostischen Einrichtungen oder Instrumenten, insbesondere Diagnose-Einrichtungen oder -instrumenten für den Mund-/Rachenraum aus der folgenden Gruppe: Spatel, Abstrich-Einrichtung, Lichtquelle, Kamera; wobei die Haltevorrichtung einen Kontaktbereich und wenigstens eine mit dem Kontaktbereich verbundene Haltekupplung aufweist. Ferner betrifft die vorliegende Erfindung die Verwendung von einer oder zwei medizintechnischen Haltevorrichtungen jeweils zum Halten von wenigstens zwei Diagnose-Einrichtungen oder -instrumenten aus der zuvor definierten Gruppe. Nicht zuletzt betrifft die vorliegende Erfindung auch eine erleichterte Dokumentation, insbesondere Befunddokumentation bei zumindest teilweise automatisierter Erfassung von Daten.

### HINTERGRUND DER ERFINDUNG

In vielen Situationen müssen sich Lebewesen vor Keimen schützen (Bakterien, Viren, Parasiten und dergleichen). Dies gilt vornehmlich für medizinische Berufsgruppen und Fachkräfte, aber auch allgemein im Alltag für jeden Mitmenschen und auch für Tiere. Zwar gibt es diverse Maßnahmen gegen Bakterien und Viren, eingeschlossen eine kontinuierliche, regelmäßige Desinfektion, jedoch lassen sich nicht jederzeit oder allerorts ausreichend effektive Maßnahmen sicherstellen. Insbesondere auch dann, wenn eine große Annäherung von Mensch zu Mensch zwecks Untersuchungen im Hals-/Rachenbereich erforderlich wird (z.B. auf dem Gebiet der hausärztlichen Versorgung, der Pädiatrie, der Dermatologie, bei den Tätigkeiten von Hals-/Nasen-/Ohrenärzten oder dergleichen), ist mit erhöhter aerogener Keimzahl (Belastung der Umgebung und der Luft) zu rechnen; dabei kann auch nicht bedenkenlos mit Desinfektionsmitteln gearbeitet werden (u.a. Inhalations-Risiko). Speziell bei Diagnostik, die auf einer Untersuchung des Rachenbereichs basieren, kann mit bisherigen Vorrichtungen nicht ausgeschlossen werden, dass sich zwei Individuen vergleichsweise nahe aneinander annähern. Herkömmliche Mundspatelhalter können die Handhabung des Spatels möglicherweise weniger riskant ausgestalten, bieten jedoch wohl keine ausreichende Sicherheit.

Die Übertragung von Keimen (Viren und dergleichen) kann auf sehr vielen unterschiedlichen Wegen erfolgen, u.a. durch Kontaktübertragung. Beispielsweise können hohe Viruslasten (bzw. Keimzahlen, Partikel-Mengen, Aerosolanteile) in der Luft (insbesondere in Aerosolen der Atemluft), an Textilien oder sonstigen Oberflächen ein großes Übertragungsrisiko mit sich bringen. Je nach Materialart und Oberflächenbeschaffenheit liegt die Persistenz der Keime bzw. Viren, insbesondere bei Raumtemperatur, bei mehreren Tagen. Ein Beispiel:
Coronaviren (SARS-CoV, MERS-CoV, SARS-CoV-2, CoVID-19) können über einen Zeitraum von deutlich über fünf Tagen, schätzungsweise sogar bis zu zehn Tagen, auf Oberflächen aus (Edel-)Metall, Glas oder Kunststoff überdauern bzw. persistieren bzw. infektiös bleiben. Materialien wie Papier, Kunststoff, Holz oder Glas scheinen dabei vergleichbar nachteilig zu sein; die Persistenz liegt dort wohl jeweils im Bereich von mindestens drei bis fünf Tagen. Mit anderen Worten: Die für die Handhabung von z.B. Spatel und Abstrich-Einrichtung genutzten Vorrichtungen oder Hilfsmittel bringen ein hohes Infektionsrisiko mit sich und müssen daher in sehr vielen Situationen häufig und sehr sorgfältig gereinigt bzw. desinfiziert werden, oder aber es muss auf Einmal-/Wegwerfprodukte zurückgegriffen werden.

Vor diesem Hintergrund besteht Interesse an innovativen Vorrichtungen, mittels welchen das virale Infektionsrisiko minimiert werden kann, beispielsweise im Vergleich zu herkömmlichen Mundspatelhaltern. Insbesondere besteht Interesse daran, möglichst nur mit solchen Vorrichtungen zu hantieren oder in Berührung zu kommen, auf welchen die Persistenz der Keime möglichst nur sehr kurz ist.

Die WO 2005/084 555 A1 beschreibt eine Vorrichtung nach dem Oberbegriff des Anspruchs 1, zur Entnahme und zum Ausstrich von Zellen und nimmt dabei auch auf Anwendungen Bezug, welche im Zusammenhang mit der vorliegenden Erfindung adressiert werden können.

Die beiden weiteren Veröffentlichungen befassen sich mit Vorrichtungen und Maßnahmen zur Untersuchung unter anderem des Rachenraums: CN 112 315 505 A, WO 2013/012 256 A2.

### ZUSAMMENFASSUNG DER ERFINDUNG

Aufgabe ist, eine Vorrichtung bereitzustellen, mittels welcher das von Keimen bzw. Viren ausgehende Infektionsrisiko für Menschen und Tiere minimiert werden kann, insbesondere im Zusammenhang mit der Handhabung von medizintechnischen Gegenständen. Die Aufgabe besteht vornehmlich auch darin, speziell für medizinische Berufsgruppen die Handhabung von medizintechnischen Gegenständen bei großer Annäherung von wenigstens zwei Lebewesen bzw. Menschen bei minimiertem viralen Infektionsrisiko zu vereinfachen, insbesondere im Zusammenhang mit Diagnosen im Mund-/Hals-/Rachenbereich. Nicht zuletzt kann die Aufgabe auch dahingehend definiert werden, dass die bereitzustellende Vorrichtung eine möglichst minimal kurze Persistenz der Keime/Viren sicherstellen kann.

Diese Aufgabe wird durch eine Vorrichtung gemäß Anspruch 1 und durch eine Verwendung gemäß dem nebengeordneten Verwendungsanspruch sowie durch den Gegenstand der nebengeordneten Ansprüche gelöst. Vorteilhafte Weiterbildungen der Erfindung werden in den jeweiligen Unteransprüchen erläutert. Die Merkmale der im Folgenden beschriebenen Ausführungsbeispiele sind miteinander kombinierbar, sofern dies nicht explizit verneint ist.

Bereitgestellt wird eine medizintechnische Haltevorrichtung eingerichtet zum Halten von allgemeinmedizinischen, chirurgischen oder diagnostischen Einrichtungen oder Instrumenten, insbesondere Diagnose-Einrichtungen oder -instrumenten für den Mund-/Rachenraum aus der folgenden Gruppe: Spatel, Abstrich-Einrichtung, Lichtquelle, Kamera, oder dergleichen diagnostische Hilfsmittel; wobei die Haltevorrichtung aufweist:
- einen Kontaktbereich zum manuellen Kontaktieren der Haltevorrichtung durch einen Nutzer (insbesondere Arzt/Ärztin);
- wenigstens eine mit dem Kontaktbereich verbundene Haltekupplung zum reversiblen Kuppeln, insbesondere form- und/oder kraftschlüssigen Kuppeln, der jeweiligen Einrichtung oder des Instruments;

Erfindungsgemäß wird vorgeschlagen, dass der Kontaktbereich eine Mindest-Längserstreckung von 20cm aufweist (bei bestimmungsgemäßer Verwendung in proximal-distal-Richtung), wobei die wenigstens eine Haltekupplung an einem Längs-Ende des Kontaktbereichs angeordnet ist (bei bestimmungsgemäßer Verwendung am aus Sicht des Nutzers distalen Ende), und wobei der Kontaktbereich zumindest teilweise aus Kupfer besteht oder Kupfer als Oberflächenmaterial/- werkstoff aufweist.

Die Erfindung basiert dabei auf zwei Konzepten und bringt diese beiden Konzepte in einer sehr einfach und schlicht ausgestalteten und leicht zu handhabenden und auch einfach herzustellenden Vorrichtung zusammen: einerseits kann bei der Handhabung und Annäherung auf einfache, leicht praktikable Weise ein Mindest-Abstand zwischen den Individuen sichergestellt werden, andererseits kann die Oberfläche der Vorrichtung bzw. die gesamte Vorrichtung desinfiziert bzw. aseptisch bzw. anti-viral ausgestaltet und bereitgestellt werden und dabei auch leicht zu pflegen bzw. leicht zu desinfizieren sein. Es hat sich auch gezeigt, dass das erfindungsgemäße Konzept in vorrichtungstechnischer Hinsicht auch eine zumindest teilweise automatisierte Massenfertigung (Serienfertigung mit großen Stückzahlen) auf vergleichsweise einfache und kostengünstige Weise ermöglicht.

Die Erfindung ermöglicht einen sicheren und nachhaltigen und verantwortungsbewussten Umgang unter Individuen, insbesondere auch dank geringer Herstellungs-/Anschaffungskosten für die Haltevorrichtung, und insbesondere auch dank einfacher Bedienung und mahnender, lehrender Handhabungshinweise, welche das Verständnis, die Sensibilität und das Bewusstsein der Nutzer bezüglich Keim- (bzw. Virus-, Bakterien-) Risiken oder sonstiger medizinischer Risiken steigern.

Es hat sich gezeigt, dass die Keimlast bzw. Virenlast dank der Kupfer-Materialauswahl nach kurzer Zeit bereits wieder sehr stark reduziert sein kann, selbst wenn die Vorrichtung in einem stark virenbelasteten Milieu eingesetzt wurde. Beispielsweise kann die Persistenz von SARS-CoV oder SARS-CoV-2 auf wenige Stunden abgesenkt werden (verminderte bakterielle und virale Erregerlast). Indem das Kupfer-Material zumindest im gesamten Kontaktbereich und wahlweise auch im Bereich der wenigstens einen Haltekupplung eingesetzt wird, kann ein antimikrobieller Effekt sichergestellt werden, welcher insbesondere auch die handhabende Person schützt (vornehmlich medizinische Fachkräfte, insbesondere den Arzt bzw. die Ärztin).

Ferner hat sich gezeigt, dass das Kupfer-Material die vorteilhaften Wirkungen insbesondere in einem feuchten Milieu verstärken kann, also dass bei hoher Aerosol-Konzentration oder hoher Luftfeuchtigkeit oder hoher relativer Feuchte auf der Kupfer-Oberfläche eine besonders gute Wirkung und damit Sicherheit für die involvierten Individuen sichergestellt werden kann.

Die vorliegende Erfindung liefert Vorteile insbesondere auch auf dem Gebiet der hausärztlichen Versorgung, der Allgemeinmedizin, inneren Medizin, Hals-/Nasen-/Ohren-Medizin, Angiologie, Dermatologie, Pädiatrie, Gynäkologie, Urologie, Hygiene, Blutspendewesen. Die Inspektion, Probenentnahme und Applikation von Medikamenten (Salben, Tinkturen, und dergleichen) sind in Kombination möglich. Dabei stehen allgemein die Körperoberflächen wie Haut und Schleimhaut im Vordergrund. Eine Anwendung erfolgt z.B. durch ein Gesundheitsamt, durch den Rettungsdienst oder die Biotaskforce der Feuerwehr, oder durch Probennahme bei Hygienekontrollen, oder bei Lebensmittelkontrollen in der Gastronomie. Eine Anwendung kann z.B. auch durch Tierärzte, Heilpraktiker und Zahnärzte erfolgen.

Die dank der erfindungsgemäßen Vorrichtung mit erhöhter Sicherheit verwendbaren Spatel können unterschiedlichen Typs sein, z.B. aus Holz, Glas, Papier/Pappe, Kunststoff, Metall.

Gemäß einem Ausführungsbeispiel ist die medizintechnische Haltevorrichtung zumindest abschnittsweise rohrförmig ist, insbesondere zumindest über 80-90% von deren Längserstreckung. Dies begünstigt nicht zuletzt auch die Reinhaltung und die mehrdimensionale Handhabung.

Gemäß einem Ausführungsbeispiel ist zumindest der Kontaktbereich, insbesondere die gesamte Haltevorrichtung, aus einem Rohr gebildet, insbesondere aus einem Kupferrohr (bevorzugt aus einem Standard-Halbzeug). Dies ermöglicht nicht zuletzt eine einfach aufgebaute und optional auch durch den Nutzer bzw. durch anwendende Ärzte oder Pflegefachkräfte individualisierbare oder für noch spezifischere Verwendungen erweiterbare Vorrichtung. Beispielsweise kann die Vorrichtung auch durch Dritte hergestellt werden, ohne dass dafür sehr viel spezifische Kenntnisse oder Ausrüstung vorhanden sein muss (minimaler Material-/Ressourceneinsatz), so dass die Vorrichtung z.B. auch in Krisensituationen einer breiten Verwendung zu Gute kommen kann.

Gemäß einem Ausführungsbeispiel ist die wenigstens eine Haltekupplung eingerichtet zum Halten einer/der Kamera, wobei die Kamera ein Kommunikationsmodul zur drahtlosen Übermittlung von Daten umfasst, insbesondere ein WLAN-Kommunikationsmodul, insbesondere mit der Kamera umfassend eine sensorisch unterstützte Ein-/Ausschalt-Automatik zum automatischen Start/Stopp der Datenerfassung und/oder -übertragung. Die Drahtlos-Kommunikation kann die (Bild-)DatenErfassung vereinfachen und effizienter ausgestalten. Die Verwendung der Haltevorrichtung wird weiter optimiert, wenn die Datenerfassung zumindest teilweise auf automatisierte Weise erfolgen kann. Dies erleichtert nicht zuletzt auch die Zuordnung von Informationen, und auch der Umfang der Informationen kann erweitert werden.

Gemäß einem Ausführungsbeispiel besteht zumindest der Kontaktbereich, insbesondere die gesamte Haltevorrichtung einschließlich der wenigstens einen Haltekupplung, aus massivem Kupfer, insbesondere unlegiert als reines Kupfer, insbesondere mit über 99% Kupferanteil, insbesondere mindestens 99,9% Kupferanteil, oder legiert mit mindestens 55%, bevorzugt mindestens 60%, weiter bevorzugt mindestens 65% oder 70% Kupferanteil. Dies liefert nicht zuletzt auch zwei wichtige Vorteile in Verbindung miteinander: Umformbarkeit zum Definieren der Geometrie der jeweiligen Haltekupplung einerseits, und gute antimikrobielle Wirkung andererseits.

Es hat sich gezeigt, dass die erfindungsgemäß realisierbaren Vorteile für viele Anwendungen besonders gut mit einem noch höheren Kupferanteil sichergestellt werden können, insbesondere mit einem Kupferanteil von über 99%, insbesondere mindestens 99,9% (bei diesem hohen Anteil kann wohl von reinem Kupfer gesprochen werden).

Gemäß einem Ausführungsbeispiel sind sowohl der Kontaktbereich als auch die wenigstens eine Haltekupplung aus einem (insbesondere einem einzigen) Kupferrohr gebildet, so dass die (jeweilige) Haltekupplung einstückig integral in den Kontaktbereich übergeht.

Gemäß einem Ausführungsbeispiel erstreckt sich die medizintechnische Haltevorrichtung in einer Haupt-Erstreckungsrichtung und ist in den weiteren beiden Raumrichtungen minimal groß ausgeführt, insbesondere in Abstimmung auf die Größen-Anforderungen an die wenigstens eine Haltekupplung. Dies erleichtert nicht zuletzt auch das Hantieren mit der Vorrichtung.

Gemäß einem Ausführungsbeispiel weist die medizintechnische Haltevorrichtung, abgesehen von der wenigstens einen Haltekupplung, einen einheitlichen Querschnitt bzw. eine einheitliche Querschnittsgeometrie auf, insbesondere eine streng zylindrische Außenmantelfläche. Dies erleichtert nicht zuletzt auch die Bedienung und kann das manuelle Greifen und Halten besonders sicher machen, insbesondere auch beim "blinden" Bedienen der Vorrichtung bzw. beim Bedienen unter Zeitdruck oder unter visuellen Einschränkungen oder Hindernissen im Sichtbereich der praktizierenden Person, also bei routinemäßigem Bedienen ganz ohne optische Kontrolle oder bei nur flüchtiger Kontrollmöglichkeit durch den Anwender.

Gemäß einem vorteilhaften Ausführungsbeispiel weist die medizintechnische Haltevorrichtung eine erste mit dem Kontaktbereich verbundene Haltekupplung und eine zweite mit dem Kontaktbereich verbundene Haltekupplung auf, wobei die Haltekupplungen an gegenüberliegenden Enden des Kontaktbereichs angeordnet sind, insbesondere jeweils mit stirnseitiger Zugänglichkeit in der Art einer Steckkupplung in Längs-Ausrichtung. Diese Ausgestaltung lässt sich auf besonders vorteilhafte Weise ausgehend von bzw. auf Basis von Kupferrohr-Halbzeugen realisieren.

Gemäß einem Ausführungsbeispiel weist die medizintechnische Haltevorrichtung wenigstens zwei Haltekupplungen auf, von denen die eine zum Kuppeln und Halten eines Spatels und die andere zum Kuppeln und Halten einer Abstrich-Einrichtung eingerichtet ist. Dies erleichtert die Arbeit insbesondere auch im Zusammenhang mit Mund-/Rachen-Untersuchungen oder Hals-/Nasen-/Ohren-Untersuchungen, und der Abstand zum jeweiligen Individuum kann vergleichsweise groß bleiben. Die Kombination mit einem Abstrich ermöglicht eine Probenentnahme zur Diagnostik. Erreger bzw. Keime werden primär über den Einsatz der Haltevorrichtung abgenommen und daraufhin sekundär über den Abstrich nachgewiesen.

Gemäß einem vorteilhaften Ausführungsbeispiel weist die medizintechnische Haltevorrichtung eine erste Haltekupplung mit einer für die form-/kraftschlüssige Aufnahme von wenigstens einem Spatel ausgebildeten ersten Kupplungs-Geometrie auf, insbesondere einer spaltförmigen und sich in Längsrichtung leicht verjüngenden Geometrie.

Gemäß einem Ausführungsbeispiel weist die medizintechnische Haltevorrichtung eine zweite Haltekupplung mit einer für die form-/kraftschlüssige Aufnahme von wenigstens einer Abstrich-Einrichtung ausgebildeten zweiten Kupplungs-Geometrie auf, insbesondere einer kreisrunden ringförmig umgrenzten Aufnahme-Geometrie.

Eine solche Kupplung liefert jeweils den Vorteil einer einfachen und schnellen Bedienung, wobei die Kupplung dabei auch auf einfache Weise an den Spatel-Typ angepasst werden kann. Die erste und zweite Haltekupplung sind bevorzugt an gegenüberliegenden freien Enden der Vorrichtung angeordnet.

Die jeweilige Haltekupplung kann insbesondere als sich nach innen verjüngende bzw. schmaler werdende Steckkupplung mit Keil-Wirkung ausgestaltet sein.

Gemäß einem vorteilhaften Ausführungsbeispiel weist die medizintechnische Haltevorrichtung eine erste Haltekupplung mit einer für die form-/kraftschlüssige Aufnahme von wenigstens einem Spatel ausgebildeten ersten Kupplungs-Geometrie auf, insbesondere einer spaltförmigen und sich in Längsrichtung leicht verjüngenden Geometrie, wobei der (Innen-)Durchmesser des Kupferrohres auf die Breite des zu haltenden Spatel-Typs für eine zumindest formschlüssige Kupplung abgestimmt ist. Dies kann eine besonders schnelle und sichere Bedienung sicherstellen. Beispielsweise bildet die Haltekupplung eine spaltförmige Aufnahme mit einer Breite, die nur 5-15% breiter ist als die Breite des Spatels, und die sich nach innen verjüngt, also kontinuierlich schmaler/kleiner wird.

Gemäß einem vorteilhaften Ausführungsbeispiel weist die medizintechnische Haltevorrichtung eine zweite Haltekupplung mit einer für die form-/kraftschlüssige Aufnahme von wenigstens einer Abstrich-Einrichtung ausgebildeten zweiten Kupplungs-Geometrie auf, insbesondere einer kreisrunden ringförmig umgrenzten Aufnahme-Geometrie, wobei der (Innen-)Durchmesser des Kupferrohres auf den Durchmesser der zu haltenden Abstrich-Einrichtung bzw. deren kuppelnden Teil oder Schaft für eine zumindest formschlüssige Kupplung abgestimmt ist. Wahlweise kann sich die Aufnahme-Geometrie nach innen verjüngen (kleiner werdender Durchmesser). Insbesondere kann die zweite Haltekupplung als Buchse zur Aufnahme eines Schaftes einer Abstrich-Einrichtung ausgestaltet sein. Wahlweise kann die Haltevorrichtung bzw. ein die Haltevorrichtung umfassendes Kit auch einen Einsatz oder eine Hülse oder einen Adapter aufweisen, welcher optional zur Größenanpassung zwischen der zweiten Haltekupplung und der Abstrich-Einrichtung angeordnet werden kann.

Es hat sich gezeigt, dass ausgehend von einem Kupferrohr auf sehr einfache Weise an einem jeweiligen freien Ende des Kupferrohres eine Steck-/Haltekupplung zum Aufnehmen eines Spatels und einer Abstrich-Einrichtung ausgestaltet werden können. Damit ist die erfindungsgemäße Haltevorrichtung auch besonders gut für eine Diagnose des Hals-/Mund-Bereiches in einer keimbelasteten Umgebung aus möglichst großer Distanz und mittels die Keime/Viren schädigenden Materialien geeignet.

An den Vorteilen hinsichtlich der Ausgestaltung der Haltekupplungen, die mit einem Rohr einhergehen, wird ersichtlich, dass die Haltevorrichtung bevorzugt aus einem Rohr hergestellt ist, insbesondere aus einem Kupferrohr-Halbzeug. Die Wandstärke kann dabei individuell gewählt werden, insbesondere derart, dass die Wandungsgeometrie (Wandform) noch durch Kaltumformen, z.B. Hämmern mit herkömmlichen im Haushalt verfügbaren (Gummi-)Hämmern, auf einfache Weise durch einen Menschen angepasst werden kann. Das Umformen kann z.B. auch durch Biegen erfolgen. Als weiterer Vorteil der rohrartigen Ausgestaltung kann ein guter Kompromiss aus Gesamtlänge und Eigengewicht genannt werden, d.h., die Handhabung wird auch bei großer Länge nicht spürbar erschwert oder beeinträchtigt, insbesondere dank eines gut austarierten Schwerpunkts bzw. dank einer vorteilhaften Position des Schwerpunkts der Vorrichtung.

Zwar kann der Kontaktbereich auch durch einen Strang aus Vollmaterial bereitgestellt werden, dieser ist jedoch schwerer, erhöht den Verbrauch des vergleichsweise teuren Kupfer-Materials, und bringt auch den Nachteil mit sich, dass die Haltekupplungen nicht so elegant an die bzw. in den freien Enden integriert werden können, insbesondere nicht einstückig, nicht spanfrei bzw. nicht ohne Einfräsungen oder ohne zusätzliche Befestigungsmittel.

Gemäß einem vorteilhaften Ausführungsbeispiel ist der Kontaktbereich im Querschnitt kreisrund ist und weist eine zylindrische Mantelfläche auf. Hierdurch kann die Handhabung und Reinhaltung verbessert werden; das Reinigen/Desinfizieren kann z.B. durch Rotation der Vorrichtung um deren Längsachse erfolgen, und das manuelle Greifen kann von allen Seiten auf dieselbe Art und Weise erfolgen.

Gemäß einem Ausführungsbeispiel ist die medizintechnische Haltevorrichtung einstückig integral aus einem Rohrabschnitt gebildet, insbesondere aus einem Kupferrohr-Halbzeug, wobei die wenigstens eine Haltekupplung und der Kontaktbereich stetig ohne Kanten oder Absätze ineinander übergehen und zusammen die Haltevorrichtung bilden. Dies begünstigt nicht zuletzt auch die Reinhaltung der Haltekupplung(en). Wahlweise kann eine Markierung eine Empfehlung für einen relativ zur Kupplung einzuhaltenden Abstand liefern.

Gemäß einem vorteilhaften Ausführungsbeispiel weist der Kontaktbereich eine Mindest-Längserstreckung von 30cm auf, und stellt dabei eine Haltegrifffläche für zwei menschliche ausgewachsene männliche oder weibliche Hände in Längsrichtung hintereinander bereit. Hierdurch kann die Variabilität/Flexibilität in der Handhabung weiter gesteigert werden, insbesondere hinsichtlich einer bidirektional verwendbaren/kuppelbaren Vorrichtung.

Gemäß einem Ausführungsbeispiel weist die medizintechnische Haltevorrichtung zumindest im Kontaktbereich einen Durchmesser im Bereich von 15mm bis 40mm auf, insbesondere 20mm bis 30mm. Dieser Bereich hat sich auch als in ergonomischer Hinsicht vorteilhaft erwiesen.

Gemäß einem Ausführungsbeispiel ist der Kontaktbereich durch eine längenspezifisch angebrachte Kennzeichnung bzw. Markierung und/oder Oberflächenbeschaffenheit gekennzeichnet bzw. in Längsrichtung begrenzt. Dies kann die Handhabung in der Praxis einfacher und sicherer ausgestalten.

Gemäß einem vorteilhaften Ausführungsbeispiel weist der Kontaktbereich eine Mindest-Längserstreckung von 30cm, 40cm oder 50cm auf. Für die Praxis hat sich für viele Anwendungen eine Länge im Bereich von 30 bis 40cm bereits als ausreichend erwiesen. Hierdurch ergibt sich ein guter Sicherheitsabstand, der bis zu 1m betragen kann (Armlänge des Arztes, Länge des Spatels, Länge der Haltekupplungen). Ab einer absoluten Länge der Haltevorrichtung von 50cm wird die Handhabung schwieriger, so dass ein guter Kompromiss bei einer absoluten Länge bis ca. 50cm sichergestellt werden kann. Dank des vergleichsweise einfachen Aufbaus der Vorrichtung ist diese auf einfache Weise in der Länge skalierbar und kann auch in einer individuell je Anwendungsfall vorteilhaften Länge bereitgestellt werden, beispielsweise exakt 42cm.

Die Erfindung wurde bisher durch Bezugnahme auf nur einen Kontaktbereich beschrieben. Die Erfindung liefert ein auf einfache Weise realisierbares Konzept, mittels welchem auch in Krisensituationen oder bei schwacher Infrastruktur oder in isolierten Lebenssituationen ein medizintechnisches Hilfsmittel realisiert werden kann, insbesondere auch durch das jeweilige Individuum bzw. den jeweiligen Nutzer selbst. In einer der einfachsten Ausgestaltungen kann die erfindungsgemäße Vorrichtung aus einem Kupferrohr selbst hergestellt werden, wobei die wenigstens eine Haltekupplung z.B. durch so genanntes Treiben (Hämmern) ausgebildet bzw. geformt werden kann (insbesondere reines Umformen, insbesondere Kaltumformen). Der (Innen-)Durchmesser des Kupferrohres ist insbesondere auf die Breite oder den Durchmesser der zu haltenden Instrumente abgestimmt, insbesondere auf die Breite eines Spatels und/oder den Durchmesser einer Abstrich-Einrichtung. Wahlweise kann das Umformen zumindest teilweise auch durch Warmumformen erfolgen.

ITEM Die zuvor genannte Aufgabe wird insbesondere auch gelöst durch eine medizintechnische Haltevorrichtung eingerichtet zum Halten von allgemeinmedizinischen, chirurgischen oder diagnostischen Einrichtungen oder Instrumenten, insbesondere Diagnose-Einrichtungen oder - instrumenten für den Mund-/Rachenraum aus der folgenden Gruppe: Spatel, Abstrich-Einrichtung, Lichtquelle, Kamera; wobei die Haltevorrichtung aufweist: einen Kontaktbereich zum manuellen Kontaktieren der Haltevorrichtung durch einen Nutzer; und wenigstens eine mit dem Kontaktbereich verbundene Haltekupplung zum reversiblen Kuppeln, insbesondere form- und/oder kraftschlüssigen Kuppeln, der jeweiligen Einrichtung oder des Instruments; wobei der Kontaktbereich eine Mindest-Längserstreckung von 20cm aufweist, wobei die wenigstens eine Haltekupplung an einem Längs-Ende des Kontaktbereichs angeordnet ist, und wobei der Kontaktbereich zumindest teilweise aus Kupfer besteht oder Kupfer als Oberflächenmaterial/-werkstoff aufweist; wobei die medizintechnische Haltevorrichtung zumindest abschnittsweise rohrförmig ist, insbesondere zumindest über 80-90% von deren Längserstreckung; wobei sowohl der Kontaktbereich als auch die wenigstens eine Haltekupplung aus einem Kupferrohr gebildet sind, so dass die Haltekupplung einstückig integral in den Kontaktbereich übergeht; wobei die medizintechnische Haltevorrichtung wenigstens zwei Haltekupplungen aufweist, von denen die eine zum Kuppeln und Halten eines/des Spatels und die andere zum Kuppeln und Halten einer/der Abstrich-Einrichtung eingerichtet ist; wobei die Haltevorrichtung einstückig integral aus einem Kupferrohr-Halbzeug gebildet ist; wobei der Kontaktbereich eine Mindest-Längserstreckung von 30cm aufweist und eine Haltegrifffläche für zwei menschliche ausgewachsene männliche oder weibliche Hände in Längsrichtung (x) hintereinander bereitstellt, wobei die wenigstens eine Haltekupplung eingerichtet ist zum Halten einer/der Kamera, wobei die Kamera ein Kommunikationsmodul zur drahtlosen Übermittlung von Daten umfasst, insbesondere mit der medizintechnischen Haltevorrichtung ferner umfassend die Kamera mit Kommunikationsmodul. Hierdurch lassen sich zahlreiche der hier zuvor beschriebenen Vorteile realisieren. Die medizintechnische Haltevorrichtung als solche wird bevorzugt ohne Kamera bereitgestellt, insbesondere um die Art der Kamera spezifisch je Anwendung wählen zu können; wahlweise kann die medizintechnische Haltevorrichtung zusammen mit der entsprechend geeigneten Kamera bereitgestellt sein/werden, insbesondere mit der Kamera in vordefinierter Längsposition gekuppelter Anordnung an der medizintechnischen Haltevorrichtung.

Die hier beschriebene Haltevorrichtung kann vorteilhaft durch die folgenden Schritte hergestellt werden.

Die Herstellung der Vorrichtung, insbesondere der ersten Kupplungs-Geometrie, kann insbesondere basierend auf vier Schritten erfolgen, ausgehend von einem Kupferrohr-Halbzeug:
1/ Einengen bzw. Abflachen im Bereich eines freien Endes des Rohres in einem Längsabstand vom freien Ende, z.B. an einer Längsposition von 3-5cm, insbesondere durch optional schrittweises Zusammendrücken; hierdurch kann auch ein Widerlager gebildet werden;
2/ weiteres Einengen bzw. Abflachen des gesamten freien Endes bis zu der zuvor definierten Längsposition, insbesondere auf eine zumindest annähernd einheitliche Dicke, z.B. im Bereich von 0,5 bis 1cm; hierdurch kann auch ein Widerlager gebildet werden;
3/ Einsetzen eines Spatels oder einer formbeständigen/formbeständigeren Spatel-Kontur in das freie Ende;
4/ weiteres Einengen bzw. Abflachen des gesamten freien Endes bis zum Kontakt der Innenmantelfläche mit dem Spatel bzw. der Spatel-Kontur;

Bzgl. der zweiten Haltekupplung ist nicht notwendigerweise ein Umform-Verfahren erforderlich; wahlweise kann das Rohr als solches bereits die Steckkupplung mit der entsprechenden Größe bereitstellen, insbesondere in Kombination mit einem Durchmesser- und/oder Form-Adapter. Jedoch sind zumindest die ersten beiden Schritte auch zum Bilden der zweiten Haltekupplung von Vorteil, insbesondere zum Bilden eines Widerlagers für den Adapter oder einen Schaft der Abstrich-Einrichtung.

Es hat sich gezeigt, dass diese Schritte einerseits auf vergleichsweise einfache Weise automatisiert werden können, andererseits auch wenig Technologie erfordern, also auch bei nur geringen Fachkenntnissen oder Fertigkeiten nachgearbeitet werden können, insbesondere auch auf improvisierte Weise bei unzureichenden Ressourcen in Not-Situationen.

Bei dem hier weiter oben und weiter unten beschriebenen Herstellungsverfahren für die hier beschriebene medizintechnische Haltevorrichtung können ausgehend von einem Kupferrohr-Halbzeug auch die folgenden Schritte vorgenommen werden:
1/ Einengen bzw. Abflachen im Bereich eines freien Endes des Rohres in einem Längsabstand vom freien Ende;
2/ weiteres Einengen bzw. Abflachen des gesamten freien Endes bis zu der zuvor definierten Längsposition;
3/ Einsetzen eines Spatels oder einer formbeständigen/formbeständigeren Spatel-Kontur in das freie Ende;
4/ weiteres Einengen bzw. Abflachen des gesamten freien Endes bis zum Kontakt der Innenmantelfläche mit dem Spatel bzw. der Spatel-Kontur. Hierdurch lassen sich hier zuvor beschriebene Vorteile realisieren.

Die zuvor genannte Aufgabe wird auch gelöst durch ein medizintechnisches Haltevorrichtungs-Kit umfassend wenigstens zwei hier zuvor beschriebene medizintechnische Haltevorrichtungen, wobei die eine (bzw. erste) der medizintechnischen Haltevorrichtungen wenigstens eine erste/zweite Haltekupplung mit einer form-/kraftschlüssigen Aufnahme für einen Spatel und/oder eine Abstrich-Einrichtung aufweist, und wobei die andere (bzw. zweite) der medizintechnischen Haltevorrichtungen wenigstens eine weitere (dritte) Haltekupplung mit einer form-/kraftschlüssigen Aufnahme für eine Lichtquelle und/oder eine Kamera aufweist, insbesondere mit dem medizintechnischen Haltevorrichtungs-Kit ferner umfassend eine/die Kamera mit Kommunikationsmodul. Dies liefert noch größere Variabilität; insbesondere kann der Fokus bzw. der Sichtbereich oder der Einsatzort (relative Position zum Individuum) sehr variabel gewählt werden.

Optional kann das Kit auch einen oder mehrere Adapter oder Hülsen oder dergleichen Zwischenstücke für eine Größenanpassung zwischen der jeweiligen Haltekupplung und der Diagnose-Einrichtung umfassen. Dies kann das Anwendungsspektrum erweitern.

Die zuvor genannte Aufgabe wird auch gelöst durch Verwendung einer hier zuvor beschriebenen medizintechnischen Haltevorrichtung zum Halten eines Spatels und/oder einer Abstrich-Einrichtung, wobei der Spatel und/oder die Abstrich-Einrichtung jeweils an einer Haltekupplung form- und/oder kraftschlüssig gekuppelt wird und dadurch in einem Längsabstand zu einem von einem Längsabschnitt der Haltevorrichtung definierten Kontaktbereich zum manuellen Kontaktieren der Haltevorrichtung durch einen Nutzer angeordnet wird, wobei der Kontaktbereich eine Mindest-Längserstreckung von 20cm aufweist, und wobei der Kontaktbereich zumindest teilweise aus Kupfer besteht oder Kupfer als Oberflächenmaterial/-werkstoff aufweist. Hierdurch lassen sich hier zuvor beschriebene Vorteile realisieren.

Die zuvor genannte Aufgabe wird auch gelöst durch Verwendung wenigstens einer hier zuvor beschriebenen medizintechnischen Haltevorrichtung, insbesondere wenigstens zweier hier zuvor beschriebener medizintechnischer Haltevorrichtungen, zum Halten eines Spatels und/oder einer Abstrich-Einrichtung und ferner eingerichtet zum Halten einer Kamera und wahlweise auch einer Lichtquelle, wobei der Spatel und/oder die Abstrich-Einrichtung jeweils an einer Haltekupplung form- und/oder kraftschlüssig gekuppelt wird und dadurch in einem Längsabstand zu einem von einem Längsabschnitt der Haltevorrichtung definierten Kontaktbereich zum manuellen Kontaktieren der Haltevorrichtung durch einen Nutzer angeordnet wird, wobei die Kamera (und wahlweise auch die Lichtquelle, welche auch in die Kamera integriert sein kann) insbesondere mittels Spann- oder Rastmechanismus gekuppelt wird, wobei der (jeweilige) Kontaktbereich eine Mindest-Längserstreckung von 20cm aufweist, und wobei der Kontaktbereich zumindest teilweise aus Kupfer besteht oder Kupfer als Oberflächenmaterial/-werkstoff aufweist, und wobei die Kamera ein Kommunikationsmodul zur drahtlosen Übermittlung von Bilddaten aufweist, wobei mittels der Kamera Bilddaten erfasst und diese mittels des Kommunikationsmoduls drahtlos übermittelt werden, insbesondere zu wenigstens einem Computer einer Arztpraxis oder eines Krankenhauses, z.B. im telemedizinischen Einsatz zur Datenübermittlung zumindest annähernd in Echtzeit. Hierdurch lassen sich hier zuvor beschriebene Vorteile realisieren.

Die mit der Vorrichtung bevorzugt reversibel kuppelbare Kamera ist z.B. als Endoskopkamera ausgestaltet, insbesondere eine Endoskopkamera mit Drahtlos-Kommunikations-Schnittstelle (Kommunikationsmodul) für Funk bzw. Internet.

Die Haltekupplung weist bevorzugt eine form-/kraftschlüssige Verjüngung des Querschnitts auf, insbesondere derart, dass eine Autoarretierung ermöglicht wird. Somit kann auch eine Abweichung des Kopplungsgegenstandes um einige Millimeter ausgeglichen werden. Wahlweise kann sich eine Verjüngung bzw. Abflachung von einem freien Ende bis zur Kupplungsstelle erstrecken, so dass die Kamera oder dergleichen Komponenten vom freien Ende bis hin zur Kupplungsstelle aufgesteckt werden können, insbesondere bei vergleichsweise großen Lagetoleranzen oder Maß-Ungenauigkeiten. Dies erleichtert/vereinfacht die Fertigung und die Verwendung noch weiter, insbesondere auch in Hinblick auf einen Austausch/Wechsel der gekuppelten Komponente.

Gemäß einem vorteilhaften Ausführungsbeispiel wird ein Kit umfassend mehrere Haltevorrichtungen und wenigstens einen Adapter zur Größen- und/oder Form-Anpassung zwischen wenigstens einer der Haltekupplungen und der entsprechenden zu haltenden Einrichtung bereitgestellt. Hierdurch kann das Anwendungsspektrum erweitert und die Variabilität bzw. Praktikabilität erhöht werden. Insbesondere kann die erforderliche Stückzahl an Vorrichtungen je Arzt, Einwohner oder Patienten minimiert werden, was z.B. auch in Krisensituationen oder bei Ressourcen-Verknappung von Vorteil ist.

Die zuvor genannte Aufgabe wird demnach auch gelöst durch ein Verfahren zum Herstellen einer medizintechnische Haltevorrichtung eingerichtet zum Halten von allgemeinmedizinischen, chirurgischen oder diagnostischen Einrichtungen oder Instrumenten, insbesondere Diagnose-Einrichtungen oder -instrumenten für den Mund-/Rachenraum aus der folgenden Gruppe: Spatel, Abstrich-Einrichtung, Lichtquelle, Kamera, oder dergleichen diagnostische Hilfsmittel, nämlich einer zuvor beschriebenen medizintechnischen Haltevorrichtung, wobei das Herstellungsverfahren zumindest die folgenden Schritte umfasst:
- Umformen, insbesondere Warmumformen eines Rohres im Bereich wenigstens eines freien Endes des Rohres, insbesondere derart dass eine Einengung bzw. Abflachung des freien Endes gebildet wird;
- Bilden eines Widerlagers durch weiteres Umformen des wenigstens einen freien Endes über einen vordefinierten Längsabschnitt, insbesondere auf eine zumindest annähernd einheitliche Dicke, z.B. im Bereich von 0,5 bis 1cm, und dadurch Ausgestalten des Widerlagers für eine Autoarretierung von Spatel und/oder Abstrich-Einrichtung;
- Ausbilden einer/der Haltekupplung der medizintechnische Haltevorrichtung zur reversiblen form-/kraftschlüssigen Montage einer/der Kamera, insbesondere indem das Rohr mit einer Verjüngung für eine Montage der Kamera in vordefinierter Längsposition versehen wird. Hierdurch lassen sich hier zuvor beschriebene Vorteile realisieren, insbesondere in Hinblick auf das einfache, kostengünstige Bereitstellen der Vorrichtung für eine Vielzahl von Anwendungen oder Nutzer.

Es hat sich gezeigt, dass in der Serienproduktion eine Warmumformung bevorzugt ist/wird, wobei wahlweise auch ein/der autoarretierende Haltemechanismus (Haltekupplung) mit Verjüngung realisierbar ist. Auch bei Implementierung einer solchen Haltekupplung kann das Eigengewicht im Verhältnis zur Gesamtlänge auf besonders vorteilhafte weise abgestimmt sein/werden.

Die vorliegende Erfindung kann auch vorteilhaft implementiert werden durch Verwendung eines Computerprogrammprodukts umfassend Befehle, die bei Ausführung des Computerprogrammproduktes auf einem Computer oder auf einem mobilen Endgerät diesen/dieses dazu veranlassen, von einer/der mittels einer zuvor beschriebenen medizintechnische Haltevorrichtung bereitgestellten Kamera erfasste (Bild-)Daten bereitzustellen, wobei das Computerprogrammprodukt eingerichtet ist zum Erfassen und Übermitteln und Archivieren oder Bereitstellen von diesen (Bild-)Daten mittels der Kamera und dem Kommunikationsmodul der medizintechnischen Haltevorrichtung. Hierdurch lassen sich hier zuvor beschriebene Vorteile insbesondere auch in Hinblick auf effizientes und mobiles Datenmanagement und Anwendung der Vorrichtung durch unterschiedliche Personen an unterschiedlichen Orten realisieren.

### KURZE BESCHREIBUNG DER FIGUREN

In den nachfolgenden Zeichnungsfiguren wird die Erfindung noch näher beschrieben, wobei für Bezugszeichen, die nicht explizit in einer jeweiligen Zeichnungsfigur beschrieben werden, auf die anderen Zeichnungsfiguren verwiesen wird. Es zeigen:
**Figur 1** in einer Draufsicht in schematischer Darstellung eine medizintechnische Haltevorrichtung gemäß einem Ausführungsbeispiel;
**Figur 2** in einer Seitenansicht in schematischer Darstellung eine medizintechnische Haltevorrichtung gemäß einem weiteren Ausführungsbeispiel;
**Figur 3** in einer Seitenansicht in schematischer Darstellung eine medizintechnische Haltevorrichtung gemäß einem weiteren Ausführungsbeispiel;
**Figur 4** in einer Seitenansicht in schematischer Darstellung ein medizintechnisches Haltevorrichtungs-Kit mit zwei medizintechnischen Haltevorrichtungen jeweils gemäß einem Ausführungsbeispiel;

### DETAILLIERTE BESCHREIBUNG DER FIGUREN

Die Figuren werden zunächst gemeinsam beschrieben. Besonderheiten werden unter Bezugnahme auf einzelne Bezugszeichen im Zusammenhang mit der jeweiligen Figur individuell erläutert.

Eine medizintechnische Haltevorrichtung 10 weist zumindest eine erste Haltekupplung 11 mit einer ersten Kupplungs-Geometrie 11a und wahlweise auch eine zweite Haltekupplung 12 mit einer zweiten Kupplungs-Geometrie 12a auf. Die erste und zweite Haltekupplung ist jeweils an einem freien Ende der Haltevorrichtung 10 angeordnet. Optional kann auch eine weitere (dritte) Haltekupplung 13 mit einer dritten Kupplungs-Geometrie 13a oder Kupplungs-Art (Befestigungsmittel) vorgesehen sein, wobei die dritte Haltekupplung 13 nicht notwendiger Weise an einem der freien Enden der Haltevorrichtung 10 angeordnet ist, sondern z.B. auch in Längsrichtung zumindest annähernd mittig in einem Kontaktbereich 15 der Haltevorrichtung 10 befestigt sein kann, insbesondere mittels form-/kraftschlüssiger Kupplung an der Oberfläche bzw. Mantelfläche 15a des Kontaktbereichs. Der Soll-Kontaktbereich 15 kann mittels einer Kennzeichnung bzw. Markierung 16 von einem durch die Haltekupplungen definierten Bereich abgegrenzt sein, insbesondere im Sinne einer Abstands-Empfehlung. Beispielsweise beträgt der Abstand zwischen dem jeweiligen freien Ende des Rohres und der Markierung 16 ca. 4cm.

Die medizintechnische Haltevorrichtung 10 ist eingerichtet, wenigstens eine Diagnose-Einrichtung aus der folgenden Gruppe mittels der jeweiligen Kupplung zu halten: Spatel 1, Abstrich-Einrichtung 2, Lichtquelle 3, Kamera 4 (insbesondere umfassend ein Kommunikationsmodul). Bevorzugt wird der Spatel 1 mittels der ersten Haltekupplung 11 gehalten, und die Abstrich-Einrichtung 2 wird mittels der zweiten Haltekupplung 12 gehalten, und die Lichtquelle 3 und/oder Kamera 4 wird mittels der dritten Haltekupplung 13 gehalten.

Ein medizintechnisches Haltevorrichtungs-Kit 20 umfasst wenigstens zwei medizintechnische Haltevorrichtungen 10, welche zusammen die drei Haltekupplungen 11, 12, 13 bereitstellen.

Die medizintechnische Haltevorrichtung 10 erstreckt sich im Wesentlichen in Längsrichtung x, wobei die Längsrichtung bei bestimmungsgemäßer Verwendung der proximal-distal-Richtung entspricht. Der Kontaktbereich 15 erstreckt sich über eine vergleichsweise große Längserstreckung x15, beispielsweise über 25 bis 35cm. In der Querrichtung y bzw. Breiten-Richtung ist die medizintechnische Haltevorrichtung 10 vergleichsweise schmal, insbesondere mit einheitlicher Breite/Durchmesser ausgestaltet, wobei bevorzugt nur eine Lichtquelle 3 und/oder Kamera 4 und gegebenenfalls auch eine/die dritte Haltekupplung 13 in Querrichtung y von der Manteloberfläche 15a hervorstehen, wobei sich die erste und zweite Haltekupplung 11, 12 bevorzugt innerhalb der konstruktiven/bauteilspezifischen Breite des Grundkörpers bzw. Rohres der Haltevorrichtung 10 befinden (also nicht darüber hinaus erstrecken). Gegebenenfalls kann die erste Haltekupplung 11 etwas breiter sein als eine Breite/Durchmesser des Kontaktbereichs 15, insbesondere wenn die erste Haltekupplung 11 durch Kaltumformen, insbesondere Treiben, abgeflacht worden ist.

Ein/das Kommunikationsmodul (insbesondere Funk-, Bluetooth- und/oder Internet-WLAN-Kommunikation), insbesondere integriert in die Kamera 4, ermöglicht eine besonders hohe örtliche Flexibilität und auch zeitliche Vorteile bei der Erfassung. Hierdurch wird nicht zuletzt auch eine Dokumentation erleichtert, insbesondere eine Befunddokumentation bei zumindest teilweise automatisierter Erfassung von (Bild-)Daten.

Ein/das Kamerasystem kann dabei spezifisch für eine jeweilige Anwendung ausgestaltet sein. Die Datenübermittlung erfolgt bevorzugt per Drahtlos-Kommunikation. Eine Schnittstell für ein jeweiliges Praxisprogramm (Computerprogramm einer Arztpraxis oder eines Krankenhauses oder dergleichen) kann als eine offene Programmierschnittstelle bereitgestellt werden, so dass die Art und Weise der Datenerfassung-/übertragung-/auswertung individuell angepasst werden kann.

Die mittels der Kamera erfassten Daten können über eine (bevorzugt offene) Programmierschnittstelle zu einem Praxis-/Krankenhaus-Managementsystem übertragen werden und z.B. in einer Patientendatenbank abgelegt werden, insbesondere zwecks Bereitstellung einer Datengrundlage für eine spätere Auswertung/Diagnose. Die Kamera soll dabei lediglich die Datenerfassung und - bereitstellung erleichtern. Eine Auswertung kann/soll durch das praktizierende Fachpersonal basierend auf den bereitgestellten Daten erleichtert werden. Somit betrifft die vorliegende Erfindung auch auf dem Konzept, das Risiko für das praktizierende Fachpersonal und die Patienten dadurch weiter zu mindern, dass die Erfassung auf effizientere Weise erfolgen kann (insbesondere bei minimierter Zeit für die Akquise von Informationen). Die Kamera bzw. das Kommunikationsmodul kann über einen Ein-/Aus-Schalter durch das praktizierende Fachpersonal für die gewünschten Momentaufnahmen hinzugeschaltet werden, insbesondere über einen gut erreichbaren Schalter der Kamera in einer auf der Haltevorrichtung montierten Anordnung der Kamera. Das Ein-/Ausschalten der Kamera kann auch automatisch erfolgen, insbesondere in Reaktion auf ein Signal betreffend die horizontale Ausrichtung der Kamera. D.h., die Kamera oder die Haltevorrichtung kann einen Neigungssensor umfassen, welcher derart eingestellt ist, dass die Datenerfassung dann ausgelöst oder unterbrochen wird, wenn die Ausrichtung/Neigung der Kamera bzw. der Haltevorrichtung wenigstens einen bestimmten Schwellwert überschreitet, beispielsweise ein Neigungssektor von ±20° in Bezug auf die Horizontale. Wahlweise kann die Haltevorrichtung auch eine Beschleunigungssensorik aufweisen, und das Ein-/Ausschalten der Kamera kann in Reaktion auf momentane Beschleunigungswerte erfolgen, z.B. indem das Ein-/Ausschalten der Kamera nur dann erfolgt, wenn die Beschleunigung einen bestimmten Schwellwert unterschreitet, also dann wenn die Haltevorrichtung vergleichsweise ruhig von einer Person gehalten wird oder abgelegt wird und nicht genutzt wird. Somit erleichtert die Erfindung nicht zuletzt auch eine effiziente/effektive Erfassung von Daten/Information bei vergleichsweise geringen Anforderungen an die Kenntnisse oder die Anzahl des Fachpersonals. Beispielsweise kann die Erfassung und das Erstellen einer Datengrundlage durch eine einzelne Person vorgenommen werden, und diese Person muss/müsste nicht notwendigerweise ein praktizierender Arzt bzw. eine praktizierende Ärztin sein (eine die (Daten-)Erfassung bzw. die Handhabung der Vorrichtung durchführende Person muss nicht notwendigerweise der/die behandelnde Arzt/Ärztin sein). Dies liefert nicht zuletzt auch in Krisensituationen Vorteile hinsichtlich Ressourcen- und Personalnutzung. Nicht zuletzt kann auch eine Rückverfolgung und ein zentralisiertes Datenmanagement erleichtert und unterstützt werden. Indem die Kamera, bevorzugt mit Kommunikationsmodul, direkt an der Haltevorrichtung in vordefinierter Ausrichtung bereitgestellt ist/wird, kann auch ein ambulante Verwendung weitgehend unabhängig von irgendwelchen medizinischen Einrichtungen erleichtert werden. Eine/die automatisierte Datenerfassung insbesondere über eine WLAN-Schnittstelle kann diesen mobilen Aspekt weiter begünstigen, so dass die erfindungsgemäße Haltevorrichtung in vielen neuen Situationen und hinsichtlich diverser Verwendungsmöglichkeiten Vorteile bietet.

Im Folgenden werden besonders vorteilhafte Ausgestaltungen der medizintechnischen Haltevorrichtung 10 bzw. des medizintechnischen Haltevorrichtungs-Kits 20 beschrieben.

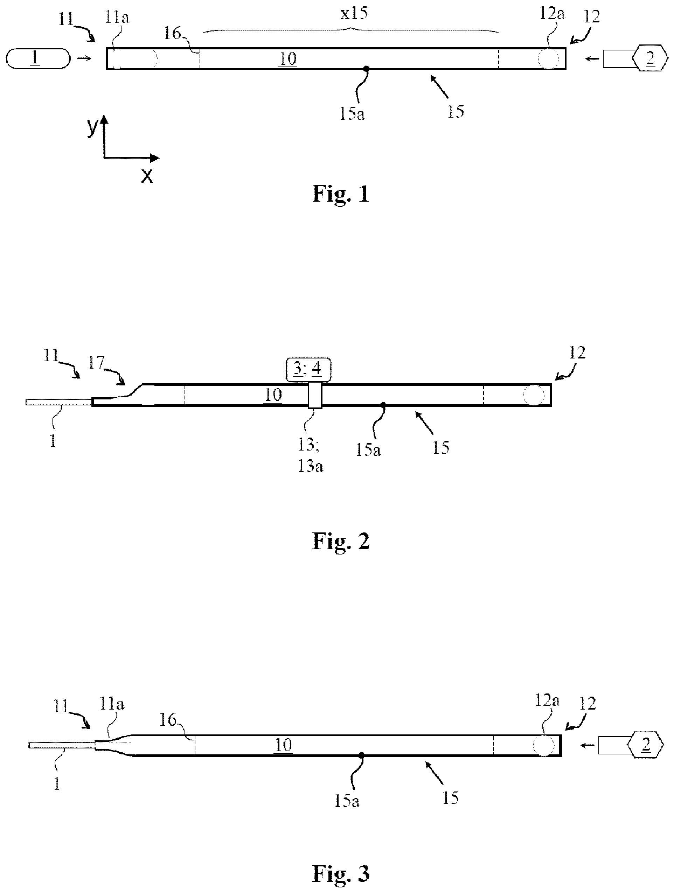

In **Fig. 1** ist eine einstückige medizintechnische Haltevorrichtung 10 illustriert, die durch ein Kupferrohr gebildet ist, wobei die beiden endseitigen Haltekupplungen 11, 12 in das Kupferrohr (Grundkörper) eingearbeitet sind, insbesondere durch Umformen und/oder durch eine Bohrung oder wahlweise auch indem ein Größenadapter vorgesehen wird. Die erste Kupplungs-Geometrie 11a ist als Rechteckquerschnitt mit abgerundeten kurzen Seiten bzw. abgerundeten Ende ausgestaltet, und die zweite Kupplungs-Geometrie 12a ist kreisrund bzw. zylindrisch ausgestaltet (Punktlinien). An der ersten Haltekupplung 11 ist ferner eine Sichtkante angedeutet (Punktlinie), ab welcher die Geometrie von der Rohrgeometrie in die Kupplungs-Geometrie übergeht, insbesondere stetig durch eine Wölbung ohne Kanten oder Absätze.

Der jeweilige Spatel 1 kann auf einfache Weise stirnseitig in Längsrichtung x in das als erste Haltekupplung 11 ausgestaltete linke freie Ende des Rohrs/Grundkörpers gesteckt werden. Die jeweilige Abstrich-Einrichtung 2 bzw. deren Schaft kann auf einfache Weise stirnseitig in bzw. entgegen der Längsrichtung x in das als zweite Haltekupplung 12 ausgestaltete rechte freie Ende des Rohrs/Grundkörpers gesteckt werden. Wahlweise kann ein Adapter oder eine Hülse oder dergleichen Zwischenstück zur Größenanpassung zwischen Abstrich-Einrichtung und Haltekupplung vorgesehen sein (insbesondere als Bestandteil eines Kits).

Die gesamte Haltevorrichtung 10 ist aus einem Kupferrohr-Halbzeug gebildet, an welchem zumindest die erste Haltekupplung 11 durch Umformen ausgebildet ist/wurde (Kalt- und/oder Warmumformen).

**Fig. 2** beschreibt eine Haltevorrichtung 10 mit zwei am jeweiligen freien Ende angeordneten Haltekupplungen 11, 12, wobei die Breite der ersten, abgeflachten Haltekupplung 11 optional auch etwas breiter sein kann (insbesondere begründet durch umformende Maßnahmen) als der Durchmesser des Kontaktbereichs 15, 15a. Bei dieser Haltevorrichtung 10 weist die erste Haltekupplung 11 eine Einengung 17 auf, die z.B. durch (Kalt-)Umformen in das Rohr bzw. in den Grundkörper (hier: entsprechend dem einstückigen Grundkörper des Kontaktbereichs 15) eingearbeitet wurde, insbesondere durch Treiben/Hämmern oder Biegen. Auch ein Warmumformen ist ergänzend oder alternativ als formgebendes Herstellungsverfahren implementierbar.

Diese Haltevorrichtung 10 umfasst zusätzlich zur ersten und zweiten Haltekupplung 11, 12 auch eine optionale dritte Haltekupplung 13 zum Halten/Kuppeln einer Lichtquelle 3 und/oder Kamera 4, wobei die relative Längsposition (x) der dritten Haltekupplung 13 am Rohr 15 optional frei wählbar ist, z.B. mittels einer kraft-/reibschlüssigen Spannkupplung.

In **Fig. 3** ist eine weitere Ausgestaltung illustriert, bei welcher die erste Haltekupplung 11 spiegelsymmetrisch ausgebildet ist und besonders zweckdienlich für einen häufigen Wechsel des Spatels ist. Wie schon erwähnt, kann die Geometrie 11a der ersten Haltekupplung 11 an die Art bzw. de Typ des Spatels 1 adaptiert sein. Beispielsweise weist die erste Haltekupplung 11 eine Breite von 2cm und eine Höhe von 0,6cm auf. Die Geometrie 11 kann z.B. durch Kaltumformen, insbesondere Biegen und/oder Treiben, in das Rohr gebracht werden. Auch ein Warmumformen ist ergänzend oder alternativ als formgebendes Herstellungsverfahren implementierbar. Beispielsweise ist das Querschnittsprofil ellipsenförmig bzw. oval. Beispielsweise weist die zweite Haltekupplung 12 einen Durchmesser von 0,8cm auf, bei zumindest annähernd kreisförmiger Geometrie. Die Tiefe/Länge der jeweiligen Haltekupplung 11, 12 beträgt z.B. 2 bis 5 cm.

Die Herstellung der Geometrie der ersten Haltekupplung 11 kann in mehreren aufeinanderfolgenden Umform-Schritten erfolgen, je nach gewünschter Ausgestaltung der Kupplung.

Die in den Fig. 2 und Fig. 3 angedeuteten geometrischen Variationen der Haltekupplung können anwendungsspezifisch gewählt werden, insbesondere auch in Hinblick auf das bevorzugte Herstellungsverfahren oder den zu haltenden Gegenstand.

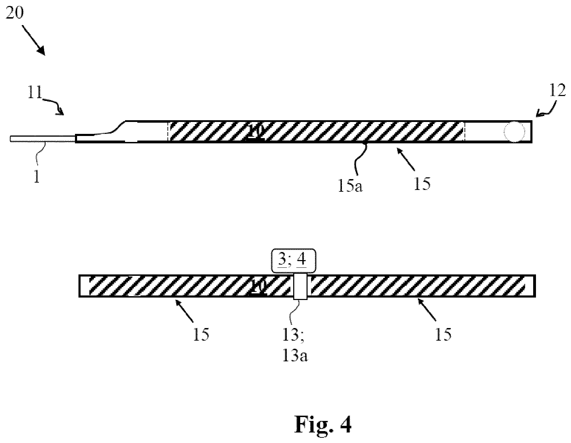

In **Fig. 4** ist ein Kit 20 umfassend zwei Haltevorrichtungen 10 gezeigt, wobei die erste Haltevorrichtung 10 ein Halten/Kuppeln von Spatel 1 und Abstrich-Einrichtung 2 mittels der ersten und zweiten Haltekupplungen 11, 12 sicherstellt, und wobei die zweite Haltevorrichtung 10 die Lichtquelle 3 und/oder Kamera 4 bereitstellt. Diese Redundanz der Haltevorrichtungen 10 hat insbesondere den Vorteil, dass die Lichtquelle 3 und/oder Kamera 4 unabhängig vom Spatel bzw. von der Abstrich-Einrichtung 2 frei im Raum angeordnet und ausgerichtet werden können. Dies kann je nach Fertigkeiten des Nutzers die Handhabung bei nur einem oder zumindest bei zwei Nutzern erleichtern. Wahlweise kann die dritte Kupplung 13 auch auf der ersten Haltevorrichtung 10 vorgesehen sein, optional redundant, um frei entscheiden zu können, ob im jeweiligen Anwendungsfall optional alle drei Kupplungen 11, 12, 13 und die entsprechenden Einrichtungen mittels nur eines Rohres 15 bereitgestellt werden sollen. Dies kann maximale Anwendungs-Variabilität sicherstellen.

Der für manuellen Kontakt durch einen Nutzer/Arzt vorgesehene Kontaktbereich 15 ist in **Fig. 3** jeweils als Schraffur illustriert. Falls auch bei der in **Fig. 3** dargestellten unteren Haltevorrichtung 10 eine oder zwei Haltekupplungen an den freien Enden vorgesehen werden sollen (insbesondere zwecks größerer Variabilität/Flexibilität), empfiehlt sich ein Mindest-Abstand zu den freien Enden, bevorzugt kenntlich gemacht durch eine Markierung oder dergleichen.

### Bezugszeichenliste

- 1: Spatel
- 2: Abstrich-Einrichtung
- 3: Lichtquelle
- 4: Kamera, insbesondere umfassend ein Kommunikationsmodul
- 10: medizintechnische Haltevorrichtung
- 11: erste Haltekupplung
- 11a: erste Kupplungs-Geometrie
- 12: zweite Haltekupplung
- 12a: zweite Kupplungs-Geometrie
- 13: weitere (dritte) Haltekupplung
- 13a: dritte Kupplungs-Geometrie
- 15: Kontaktbereich
- 15a: Oberfläche bzw. Mantelfläche des Kontaktbereichs
- 16: Kennzeichnung bzw. Markierung
- 17: Einengung

- 20: Medizintechnisches Haltevorrichtungs-Kit

- x: Längsrichtung bzw. proximal-distal-Richtung (bei bestimmungsgemäßer Verwendung)
- x15: Längserstreckung Kontaktbereich
- y: Querrichtung bzw. Breiten-Richtung

## Patentansprüche

1. Medizintechnische Haltevorrichtung (10) eingerichtet zum Halten von allgemeinmedizinischen, chirurgischen oder diagnostischen Einrichtungen oder Instrumenten, insbesondere Diagnose-Einrichtungen oder -instrumenten für den Mund-/Rachenraum aus der folgenden Gruppe: Spatel (1), Abstrich-Einrichtung (2), Lichtquelle (3), Kamera (4); wobei die Haltevorrichtung (10) aufweist:
- einen Kontaktbereich (15) zum manuellen Kontaktieren der Haltevorrichtung (10) durch einen Nutzer;
- wenigstens eine mit dem Kontaktbereich (15) verbundene Haltekupplung (11, 12, 13) zum reversiblen Kuppeln, insbesondere form- und/oder kraftschlüssigen Kuppeln, der jeweiligen Einrichtung oder des Instruments;
wobei der Kontaktbereich (15) eine Mindest-Längserstreckung (x15) von 20cm aufweist, wobei die wenigstens eine Haltekupplung (11, 12) an einem Längs-Ende des Kontaktbereichs (15) angeordnet ist, und **dadurch gekennzeichnet, dass**
der Kontaktbereich (15) zumindest teilweise aus Kupfer besteht oder Kupfer als Oberflächenmaterial/-werkstoff aufweist.

2. Medizintechnische Haltevorrichtung (10) nach Anspruch 1, wobei die medizintechnische Haltevorrichtung (10) zumindest abschnittsweise rohrförmig ist, insbesondere zumindest über 80-90% von deren Längserstreckung; oder wobei der Kontaktbereich (15) im Querschnitt kreisrund ist und eine zylindrische Mantelfläche (15a) aufweist.; oder wobei zumindest der Kontaktbereich (15), insbesondere die gesamte Haltevorrichtung, aus einem Rohr gebildet ist, insbesondere aus einem Kupferrohr; oder wobei die medizintechnische Haltevorrichtung (10) einstückig integral aus einem Rohrabschnitt gebildet ist, insbesondere aus einem Kupferrohr-Halbzeug, wobei die wenigstens eine Haltekupplung (11, 12) und der Kontaktbereich (15) stetig ohne Kanten oder Absätze ineinander übergehen und zusammen die Haltevorrichtung (10) bilden.

3. Medizintechnische Haltevorrichtung (10) nach einem der vorhergehenden Ansprüche, wobei die wenigstens eine Haltekupplung (11, 12) eingerichtet ist zum Halten einer/der Kamera (4), wobei die Kamera ein Kommunikationsmodul zur drahtlosen Übermittlung von Daten umfasst, insbesondere ein WLAN-Kommunikationsmodul, insbesondere mit der Kamera umfassend eine sensorisch unterstützte Ein-/Ausschalt-Automatik zum automatischen Start/Stopp der Datenerfassung und/oder - übertragung.

4. Medizintechnische Haltevorrichtung (10) nach einem der vorhergehenden Ansprüche, wobei zumindest der Kontaktbereich (15), insbesondere die gesamte Haltevorrichtung einschließlich der wenigstens einen Haltekupplung (11, 12), aus massivem Kupfer besteht, insbesondere unlegiert als reines Kupfer, insbesondere mit über 99% Kupferanteil, insbesondere mindestens 99,9% Kupferanteil, oder legiert mit mindestens 55%, bevorzugt mindestens 60%, weiter bevorzugt mindestens 65% oder 70% Kupferanteil; oder wobei sowohl der Kontaktbereich (15) als auch die wenigstens eine Haltekupplung (11, 12) aus einem Kupferrohr gebildet sind, so dass die Haltekupplung (11, 12) einstückig integral in den Kontaktbereich übergeht.

5. Medizintechnische Haltevorrichtung (10) nach einem der vorhergehenden Ansprüche, wobei die medizintechnische Haltevorrichtung (10) eine erste mit dem Kontaktbereich (15) verbundene Haltekupplung (11) aufweist und eine zweite mit dem Kontaktbereich verbundene Haltekupplung (12) aufweist, wobei die Haltekupplungen (11, 12) an gegenüberliegenden Enden des Kontaktbereichs (15) angeordnet sind, insbesondere jeweils mit stirnseitiger Zugänglichkeit in der Art einer Steckkupplung in Längs-Ausrichtung; oder wobei die medizintechnische Haltevorrichtung (10) wenigstens zwei Haltekupplungen (11, 12) aufweist, von denen die eine zum Kuppeln und Halten eines/des Spatels (1) und die andere zum Kuppeln und Halten einer/der Abstrich-Einrichtung (2) eingerichtet ist.

6. Medizintechnische Haltevorrichtung (10) nach einem der vorhergehenden Ansprüche, wobei die medizintechnische Haltevorrichtung (10) eine erste Haltekupplung (11) mit einer für die form-/kraftschlüssige Aufnahme von wenigstens einem Spatel (1) ausgebildeten ersten Kupplungs-Geometrie aufweist, insbesondere einer spaltförmigen und sich in Längsrichtung leicht verjüngenden Geometrie; oder wobei die medizintechnische Haltevorrichtung (10) eine zweite Haltekupplung (12) mit einer für die form-/kraftschlüssige Aufnahme von wenigstens einer Abstrich-Einrichtung (2) ausgebildeten zweiten Kupplungs-Geometrie aufweist, insbesondere einer kreisrunden ringförmig umgrenzten Aufnahme-Geometrie.

7. Medizintechnische Haltevorrichtung (10) nach einem der vorhergehenden Ansprüche, wobei die medizintechnische Haltevorrichtung (10) eine erste Haltekupplung (11) mit einer für die form-/kraftschlüssige Aufnahme von wenigstens einem Spatel (1) ausgebildeten ersten Kupplungs-Geometrie aufweist, insbesondere einer spaltförmigen und sich in Längsrichtung leicht verjüngenden Geometrie, wobei der (Innen-)Durchmesser des Kupferrohres auf die Breite des zu haltenden Spatel-Typs für eine zumindest formschlüssige Kupplung abgestimmt ist; oder wobei die medizintechnische Haltevorrichtung (10) eine zweite Haltekupplung (12) mit einer für die form-/kraftschlüssige Aufnahme von wenigstens einer Abstrich-Einrichtung (2) ausgebildeten zweiten Kupplungs-Geometrie aufweist, insbesondere einer kreisrunden ringförmig umgrenzten Aufnahme-Geometrie, wobei der (Innen-)Durchmesser des Kupferrohres auf den Durchmesser der zu haltenden Abstrich-Einrichtung (2) bzw. deren kuppelnden Teil oder Schaft für eine zumindest formschlüssige Kupplung abgestimmt ist.

8. Medizintechnische Haltevorrichtung (10) nach einem der vorhergehenden Ansprüche, wobei der Kontaktbereich (15) eine Mindest-Längserstreckung von 30cm aufweist und eine Haltegrifffläche für zwei menschliche ausgewachsene männliche oder weibliche Hände in Längsrichtung (x) hintereinander bereitstellt.

9. Medizintechnische Haltevorrichtung (10) nach einem der vorhergehenden Ansprüche, wobei der Kontaktbereich (15) durch eine längenspezifisch angebrachte Kennzeichnung bzw. Markierung (16) und/oder Oberflächenbeschaffenheit gekennzeichnet bzw. in Längsrichtung (x) begrenzt ist; oder wobei der Kontaktbereich (15) eine Mindest-Längserstreckung (x) von 30cm, 40cm oder 50cm aufweist.

10. Medizintechnische Haltevorrichtung (10) nach Anspruch 1, wobei die medizintechnische Haltevorrichtung (10) zumindest abschnittsweise rohrförmig ist, insbesondere zumindest über 80-90% von deren Längserstreckung; wobei sowohl der Kontaktbereich (15) als auch die wenigstens eine Haltekupplung (11, 12) aus einem Kupferrohr gebildet sind, so dass die Haltekupplung (11, 12) einstückig integral in den Kontaktbereich übergeht; wobei die medizintechnische Haltevorrichtung (10) wenigstens zwei Haltekupplungen (11, 12) aufweist, von denen die eine zum Kuppeln und Halten eines/des Spatels (1) und die andere zum Kuppeln und Halten einer/der Abstrich-Einrichtung (2) eingerichtet ist; wobei die Haltevorrichtung (10) einstückig integral aus einem Kupferrohr-Halbzeug gebildet ist; wobei der Kontaktbereich (15) eine Mindest-Längserstreckung von 30cm aufweist und eine Haltegrifffläche für zwei menschliche ausgewachsene männliche oder weibliche Hände in Längsrichtung (x) hintereinander bereitstellt; wobei die wenigstens eine Haltekupplung (11, 12) eingerichtet ist zum Halten einer/der Kamera (4), wobei die Kamera ein Kommunikationsmodul zur drahtlosen Übermittlung von Daten umfasst, insbesondere mit der medizintechnischen Haltevorrichtung (10) ferner umfassend die Kamera mit Kommunikationsmodul.

11. Medizintechnisches Haltevorrichtungs-Kit (20) umfassend wenigstens zwei medizintechnische Haltevorrichtungen (10) jeweils nach einem der vorhergehenden Ansprüche, wobei die eine der medizintechnischen Haltevorrichtungen (10) wenigstens eine erste/zweite Haltekupplung (11, 12) mit einer form-/kraftschlüssigen Aufnahme für einen/den Spatel (1) und/oder eine/die Abstrich-Einrichtung (2) aufweist, und wobei die andere der medizintechnischen Haltevorrichtungen (10) wenigstens eine weitere Haltekupplung (13) mit einer form-/kraftschlüssigen Aufnahme für eine/die Lichtquelle (3) und/oder eine/die Kamera (4) aufweist, insbesondere mit dem medizintechnischen Haltevorrichtungs-Kit ferner umfassend eine/die Kamera mit Kommunikationsmodul.

12. Verwendung einer medizintechnischen Haltevorrichtung (10) nach einem der Ansprüche 1 bis 10 zum Halten eines Spatels (1) und/oder einer Abstrich-Einrichtung (2), wobei der Spatel und/oder die Abstrich-Einrichtung jeweils an einer Haltekupplung (11, 12) form- und/oder kraftschlüssig gekuppelt wird und dadurch in einem Längsabstand (x) zu einem von einem Längsabschnitt der Haltevorrichtung definierten Kontaktbereich (x15) zum manuellen Kontaktieren der Haltevorrichtung durch einen Nutzer angeordnet wird.

13. Verwendung wenigstens einer medizintechnischen Haltevorrichtung (10) nach Anspruch 3, oder nach einem der Ansprüche 4-9, insofern abhängig von Anspruch 3, oder nach Anspruch 10, wobei der Spatel und/oder die Abstrich-Einrichtung jeweils an einer Haltekupplung (11, 12) form- und/oder kraftschlüssig gekuppelt wird und dadurch in einem Längsabstand (x) zu einem von einem Längsabschnitt der Haltevorrichtung definierten Kontaktbereich (x15) zum manuellen Kontaktieren der Haltevorrichtung (10) durch einen Nutzer angeordnet wird, wobei die Kamera (4) insbesondere mittels Spann- oder Rastmechanismus gekuppelt wird, und wobei die Kamera (4) ein Kommunikationsmodul zur drahtlosen Übermittlung von Bilddaten aufweist, wobei mittels der Kamera Bilddaten erfasst und diese mittels des Kommunikationsmoduls drahtlos übermittelt werden, insbesondere zu wenigstens einem Computer einer Arztpraxis oder eines Krankenhauses.

14. Verfahren zum Herstellen einer medizintechnische Haltevorrichtung (10) eingerichtet zum Halten von allgemeinmedizinischen, chirurgischen oder diagnostischen Einrichtungen oder Instrumenten, insbesondere Diagnose-Einrichtungen oder -instrumenten für den Mund-/Rachenraum aus der folgenden Gruppe: Spatel, Abstrich-Einrichtung, Lichtquelle, Kamera, oder dergleichen diagnostische Hilfsmittel, nämlich einer medizintechnischen Haltevorrichtung nach einem der Vorrichtungsansprüche 1 bis 10, wobei das Herstellungsverfahren zumindest die folgenden Schritte umfasst:
- Umformen, insbesondere Warmumformen eines Rohres im Bereich wenigstens eines freien Endes des Rohres, insbesondere derart dass eine Einengung bzw. Abflachung des freien Endes gebildet wird;
- Bilden eines Widerlagers durch weiteres Umformen des wenigstens einen freien Endes über einen vordefinierten Längsabschnitt, insbesondere auf eine zumindest annähernd einheitliche Dicke, z.B. im Bereich von 0,5 bis 1cm, und dadurch Ausgestalten des Widerlagers für eine Autoarretierung von Spatel und/oder Abstrich-Einrichtung;
- Ausbilden einer/der Haltekupplung der medizintechnische Haltevorrichtung (10) zur reversiblen form-/kraftschlüssigen Montage einer/der Kamera, insbesondere indem das Rohr mit einer Verjüngung für eine Montage der Kamera in vordefinierter Längsposition versehen wird.

15. Verfahren nach dem vorhergehenden Anspruch, wobei ausgehend von einem Kupferrohr-Halbzeug die folgenden Schritte vorgenommen werden:
1/ Einengen bzw. Abflachen im Bereich eines freien Endes des Rohres in einem Längsabstand vom freien Ende;
2/ weiteres Einengen bzw. Abflachen des gesamten freien Endes bis zu der zuvor definierten Längsposition;
3/ Einsetzen eines Spatels oder einer formbeständigen/formbeständigeren Spatel-Kontur in das freie Ende;
4/ weiteres Einengen bzw. Abflachen des gesamten freien Endes bis zum Kontakt der Innenmantelfläche mit dem Spatel bzw. der Spatel-Kontur.

## Claims

1. Medical holding device (10) for holding general medical, surgical or diagnostic devices or instruments, in particular diagnostic devices or instruments for the oral/pharyngeal cavity from the following group: spatula (1), swab device (2), light source (3), camera (4); wherein the holding device (10) comprises:
- a contact area (15) for manual contact with the holding device (10) by a user;
- at least one holding coupling (11, 12, 13) connected to the contact area (15) for reversibly coupling, in particular for form- and/or force-fitting coupling, the respective device or instrument,
wherein the contact area (15) has a minimum longitudinal extent (x15) of 20 cm, wherein the at least one holding coupling (11, 12) is arranged at a longitudinal end of the contact area (15);
**characterised in that** the contact area (15) consists at least partly of copper or comprises copper as a surface material.

2. Medical holding device (10) according to claim 1, wherein the medical holding device (10) is tubular at least in sections, in particular over at least 80-90% of its longitudinal extent; or wherein the contact area (15) has a circular cross-section and comprises a cylindrical outer surface (15a); or wherein at least the contact area (15), in particular the entire holding device, is formed from a tube, in particular from a copper tube; or wherein the medical holding device (10) is formed integrally in one piece from a tubular section, in particular from a semi-finished copper tube, wherein the at least one holding coupling (11, 12) and the contact area (15) merge seamlessly into one another without edges or steps and together form the holding device (10).

3. Medical holding device (10) according to one of the preceding claims, wherein the at least one holding coupling (11, 12) is configured to hold a/the camera (4), wherein the camera comprises a communication module for the wireless transmission of data, in particular a Wi-Fi communication module, in particular comprising, together with the camera, a sensor-assisted automatic on/off function for the automatic start/stop of data acquisition and/or transmission.

4. Medical holding device (10) according to any one of the preceding claims, wherein at least the contact area (15), in particular the entire holding device including the at least one holding coupling (11, 12), is made of solid copper, in particular unalloyed pure copper, in particular with a copper content of over 99%, in particular at least 99.9% copper, or alloyed with at least 55%, preferably at least 60%, more preferably at least 65% or 70% copper; or wherein both the contact area (15) and the at least one holding coupling (11, 12) are formed from a copper tube, such that the holding coupling (11, 12) merges integrally into the contact area in a single piece.

5. Medical holding device (10) according to any one of the preceding claims, wherein the medical holding device (10) comprises a first holding coupling (11) connected to the contact area (15) and a second holding coupling (12) connected to the contact area, wherein the holding couplings (11, 12) are arranged at opposite ends of the contact area (15), in particular each being accessible from the front in the manner of a plug-in coupling in a longitudinal orientation; or wherein the medical holding device (10) comprises at least two holding couplings (11, 12), one of which is configured to couple and retain a/the spatula (1) and the other to couple and retain a/the swab device (2).

6. Medical holding device (10) according to any one of the preceding claims, wherein the medical holding device (10) comprises a first holding coupling (11) with a first coupling geometry formed for the form- and/or force-fitting reception of at least one spatula (1), in particular a slit-shaped geometry tapering slightly in the longitudinal direction; or wherein the medical holding device (10) comprises a second holding coupling (12) with a second coupling geometry designed for the form- and/or force-fitting reception of at least one swab device (2), in particular a circular, ring-shaped receptacle geometry.

7. Medical holding device (10) according to any one of the preceding claims, wherein the medical holding device (10) comprises a first holding coupling (11) with a first coupling geometry designed for the form- and/or force-fitting reception of at least one spatula (1), in particular a slit-shaped geometry tapering slightly in the longitudinal direction, wherein the (inner) diameter of the copper tube is matched to the width of the spatula type to be held to ensure at least a form-fit coupling; or wherein the medical holding device (10) comprises a second holding coupling (12) with a second coupling geometry designed for the form- and/or force-fitting reception of at least one swab device (2), in particular a circular, ring-shaped receptacle geometry, wherein the (inner) diameter of the copper tube is adapted to the diameter of the swab device (2) to be held, or its coupling part or shaft, for at least a form-fit coupling.

8. Medical holding device (10) according to one of the preceding claims, wherein the contact area (15) has a minimum longitudinal extent of 30 cm and provides a holding grip surface for two adult human male or female hands placed one behind the other in the longitudinal direction (x).

9. Medical holding device (10) according to any one of the preceding claims, wherein the contact area (15) is **characterised by** a length-specific labelling or marking (16) and/or surface texture and/or the contact area is thereby delimited in the longitudinal direction (x); or wherein the contact area (15) has a minimum longitudinal extent (x) of 30 cm, 40 cm or 50 cm.

10. Medical holding device (10) according to claim 1, wherein the medical holding device (10) is tubular at least in sections, in particular over at least 80-90% of its longitudinal extent; wherein both the contact area (15) and the at least one holding coupling (11, 12) are formed from a copper tube, such that the holding coupling (11, 12) merges integrally into the contact area; wherein the medical holding device (10) comprises at least two holding couplings (11, 12), one of which is configured to couple and hold a/the spatula (1) and the other to couple and hold a/the swab device (2); wherein the holding device (10) is formed integrally from a semi-finished copper tube; wherein the contact area (15) has a minimum longitudinal extent of 30 cm and provides a gripping surface for two adult human male or female hands placed one behind the other in the longitudinal direction (x); wherein the at least one holding coupling (11, 12) is configured to hold a/the camera (4), wherein the camera comprises a communication module for the wireless transmission of data, in particular with the medical holding device (10) further including the camera with communication module.

11. Medical holding device kit (20) comprising at least two medical holding devices (10), each according to one of the preceding claims, wherein one of the medical holding devices (10) comprises at least a first/second holding coupling (11, 12) with a form- and/or force-fitting receptacle for a/the spatula (1) and/or a/the swab device (2), and wherein the other of the medical holding devices (10) comprises at least one further holding coupling (13) with a form- and/or force-fitting receptacle for a/the light source (3) and/or a/the camera (4), in particular with the medical holding device kit further comprising a/the camera with communication module.

12. Use of a medical holding device (10) according to any one of claims 1 to 10 for holding a spatula (1) and/or a swab device (2), wherein the spatula and/or the swab device is coupled to a holding coupling (11, 12) by form- and/or force-fitting, and is thereby arranged at a longitudinal distance (x) from a contact area (x15) defined by a longitudinal section of the holding device for manual contact with the holding device by a user.

13. Use of at least one medical holding device (10) according to claim 3, or according to any one of claims 4 to 9, insofar as dependent on claim 3, or according to claim 10, wherein the spatula and/or the swab device is respectively coupled by form- and/or force-fitting to a holding coupling (11, 12) and is thereby arranged at a longitudinal distance (x) from a contact area (x15) defined by a longitudinal section of the holding device for manual contact with the holding device (10) by a user, wherein the camera (4) is coupled in particular by means of a clamping or locking mechanism, and wherein the camera (4) comprises a communication module for the wireless transmission of image data, wherein image data is captured by means of the camera and transmitted wirelessly by means of the communication module, in particular to at least one computer in a doctor's surgery or a hospital.

14. Method for manufacturing a medical holding device (10) configured to hold general medical, surgical or diagnostic devices or instruments, in particular diagnostic devices or instruments for the oral/pharyngeal cavity from the following group: spatulas, swab devices, light sources, cameras, or similar diagnostic aids, namely a medical holding device according to any one of claims 1 to 10, wherein the manufacturing method comprises at least the following steps:
- forming, in particular hot forming, of a tube in the region of at least one free end of the tube, in particular such that a constriction or flattening of the free end is formed;
- forming an abutment by further forming the at least one free end over a predefined longitudinal section, in particular to an at least approximately uniform thickness, e.g. in the range of 0.5 to 1 cm, and thereby configuring the abutment for self-locking of the spatula and/or swab device;
- forming a holding coupling of the medical holding device (10) for the reversible form- and/or force-fit mounting of a camera, in particular by providing the tube with a taper for mounting the camera in a predefined longitudinal position.

15. Method according to the preceding claim, wherein, starting from a semi-finished copper tube, the following steps are carried out:
1/ constricting or flattening in the region of a free end of the tube at a longitudinal distance from the free end;
2/ further constricting or flattening the entire free end up to the previously defined longitudinal position;
3/ inserting into the free end a spatula or a spatula contour which is dimensionally stable or even more dimensionally stable;
4/ further constricting or flattening the entire free end until the inner surface comes into contact with the spatula or the spatula contour.

## Revendications

1. Dispositif de maintien de la technique médicale (10) conçu pour maintenir des dispositifs ou des instruments médicaux généraux, chirurgicaux ou diagnostiques, en particulier des dispositifs ou des instruments de diagnostic pour la cavité buccale et le pharynx, parmi le groupe suivant : spatule (1), dispositif de prélèvement (2), source lumineuse (3), caméra (4) ; le dispositif de maintien (10) comportant :
- une zone de contact (15) permettant à un utilisateur d'entrer en contact manuellement avec le dispositif de maintien (10) ;
- au moins un raccord de fixation (11, 12, 13) relié à la zone de contact (15) pour le raccordement réversible, en particulier par complémentarité de forme et/ou par adhérence, du dispositif ou de l'instrument concerné,
la zone de contact (15) présentant une extension longitudinale minimale (x15) de 20 cm, l'au moins un raccord de fixation (11, 12) étant disposé à une extrémité longitudinale de la zone de contact (15) ;
**caractériséence que** la zone de contact (15) est constituée au moins en partie de cuivre ou comporte du cuivre comme matériau de surface.

2. Dispositif de maintien de la technique médicale (10) selon la revendication 1, dans lequel le dispositif de maintien de la technique médicale (10) est tubulaire au moins par sections, en particulier sur au moins 80 à 90 % de sa longueur ; ou dans lequel la zone de contact (15) présente une section transversale circulaire et comporte une surface d'enveloppe cylindrique (15a) ; ou dans lequel au moins la zone de contact (15), en particulier l'ensemble du dispositif de maintien, est formée d'un tube, en particulier d'un tube en cuivre ; ou dans lequel le dispositif de maintien de la technique médicale (10) est formé d'un seul tenant à partir d'un segment de tube, en particulier à partir d'un produit semi-fini en tube de cuivre, le ou les raccords de fixation (11, 12) et la zone de contact (15) se raccordant de manière continue, sans arêtes ni décrochements, et formant ensemble le dispositif de maintien (10).

3. Dispositif de maintien de la technique médicale (10) selon l'une des revendications précédentes, dans lequel le ou les raccords de fixation (11, 12) sont conçus pour maintenir une/la caméra (4), la caméra comprenant un module de communication pour la transmission sans fil de données, en particulier un module de communication Wi-Fi, notamment avec la caméra comprenant un système de mise en marche/arrêt automatique assisté par capteurs pour le démarrage/l'arrêt automatique de l'acquisition et/ou de la transmission de données.

4. Dispositif de maintien de la technique médicale (10) selon l'une des revendications précédentes, dans lequel au moins la zone de contact (15), en particulier l'ensemble du dispositif de maintien y compris le ou les raccords de fixation (11, 12), est constituée de cuivre massif, en particulier de cuivre pur non allié, en particulier avec une teneur en cuivre supérieure à 99 %, en particulier d'au moins 99,9 % de cuivre, ou alliée avec une teneur en cuivre d'au moins 55 %, de préférence d'au moins 60 %, et de manière encore plus préférée d'au moins 65 % ou 70 % ; ou dans lequel tant la zone de contact (15) que le ou les raccords de fixation (11, 12) sont formés à partir d'un tube de cuivre, de sorte que le raccord de fixation (11, 12) se prolonge d'un seul tenant dans la zone de contact.

5. Dispositif de maintien de la technique médicale (10) selon l'une des revendications précédentes, le dispositif de maintien de la technique médicale (10) comportant un premier raccord de fixation (11) relié à la zone de contact (15) et un deuxième raccord de fixation (12) relié à la zone de contact, les raccords de fixation (11, 12) sont disposés aux extrémités opposées de la zone de contact (15), en particulier avec une accessibilité frontale respective à la manière d'un raccord enfichable dans une orientation longitudinale ; ou dans lequel le dispositif de maintien de la technique médicale (10) comporte au moins deux raccords de fixation (11, 12), dont l'un est conçu pour raccorder et maintenir une/la spatule (1) et l'autre pour raccorder et maintenir un/le dispositif de prélèvement (2).

6. Dispositif de maintien de la technique médicale (10) selon l'une des revendications précédentes, le dispositif de maintien de la technique médicale (10) comportant un premier raccord de fixation (11) présentant une première géométrie de raccord conçue pour recevoir par complémentarité de forme et par adhérence au moins une spatule (1), en particulier une géométrie en forme de fente et se rétrécissant légèrement dans le sens longitudinal ; ou dans lequel le dispositif de maintien de la technique médicale (10) comporte un deuxième raccord de fixation (12) présentant une deuxième géométrie de raccord conçue pour recevoir par complémentarité de forme et par adhérence au moins un dispositif de prélèvement (2), en particulier une géométrie de logement circulaire délimitée par un anneau.

7. Dispositif de maintien de la technique médicale (10) selon l'une des revendications précédentes, dans lequel le dispositif de maintien de la technique médicale (10) comporte un premier raccord de fixation (11) avec une première géométrie de raccord conçue pour recevoir par complémentarité de forme et/ou par adhérence de force au moins une spatule (1), en particulier une géométrie en forme de fente et se rétrécissant légèrement dans le sens longitudinal, le diamètre (intérieur) du tube de cuivre étant adapté à la largeur du type de spatule à maintenir pour un accouplement au moins par complémentarité de forme ; ou dans lequel le dispositif de maintien de la technique médicale (10) comporte un deuxième raccord de fixation (12) présentant une deuxième géométrie de raccord conçue pour recevoir par complémentarité de forme et par adhérence au moins un dispositif de prélèvement (2), en particulier une géométrie de logement circulaire délimitée par un anneau, le diamètre (intérieur) du tube en cuivre est adapté au diamètre du dispositif de prélèvement (2) à maintenir ou de sa partie d'accouplement ou de sa tige pour un accouplement au moins par complémentarité de forme.

8. Dispositif de maintien de la technique médicale (10) selon l'une des revendications précédentes, la zone de contact (15) présentant une extension longitudinale minimale de 30 cm et offrant une surface de préhension permettant à deux mains humaines adultes, masculines ou féminines, de se placer l'une derrière l'autre dans le sens longitudinal (x).

9. Dispositif de maintien de la technique médicale (10) selon l'une des revendications précédentes, dans lequel la zone de contact (15) est **caractérisée par** un repère ou un marquage (16) apposé à une longueur spécifique et/ou par une texture de surface, ou est délimitée dans le sens longitudinal (x) ; ou dans lequel la zone de contact (15) présente une extension longitudinale minimale (x) de 30 cm, 40 cm ou 50 cm.

10. Dispositif de maintien de la technique médicale (10) selon la revendication 1, dans lequel le dispositif de maintien de la technique médicale (10) est tubulaire au moins par sections, en particulier sur au moins 80 à 90 % de son extension longitudinale ; la zone de contact (15) ainsi que le ou les raccords de fixation (11, 12) étant formés à partir d'un tube en cuivre, de sorte que le raccord de fixation (11, 12) se prolonge d'un seul tenant dans la zone de contact ; le dispositif de maintien de la technique médicale (10) comportant au moins deux raccords de fixation (11, 12), dont l'un est conçu pour raccorder et maintenir une/la spatule (1) et l'autre pour raccorder et maintenir un/le dispositif de prélèvement (2) ; le dispositif de maintien (10) étant formé d'un seul tenant à partir d'un produit semi-fini en tube de cuivre ; la zone de contact (15) présentant une extension longitudinale minimale de 30 cm et offrant une surface de préhension pour deux mains humaines adultes, masculines ou féminines, disposées l'une derrière l'autre dans le sens longitudinal (x) ; au moins un des raccords de fixation (11, 12) étant conçu pour maintenir une/la caméra (4), la caméra comprenant un module de communication pour la transmission sans fil de données, en particulier avec le dispositif de maintien de la technique médicale (10) comprenant en outre la caméra avec module de communication.

11. Kit de dispositifs de maintien de la technique médicale (20) comprenant au moins deux dispositifs de maintien de la technique médicale (10) selon l'une des revendications précédentes, l'un des dispositifs de maintien de la technique médicale (10) comportant au moins un premier/deuxième raccord de fixation (11, 12) avec un logement par complémentarité de forme et par adhérence pour une/la spatule (1) et/ou un/le dispositif de prélèvement (2), et l'autre des dispositifs de maintien de la technique médicale (10) comportant au moins un autre raccord de fixation (13) avec un logement par complémentarité de forme et par adhérence pour une/la source lumineuse (3) et/ou une/la caméra (4), le kit de dispositifs de maintien de la technique médicale comprenant en outre une/la caméra avec module de communication.

12. Utilisation d'un dispositif de maintien de la technique médicale (10) selon l'une des revendications 1 à 10 pour maintenir une spatule (1) et/ou un dispositif de prélèvement (2), la spatule et/ou le dispositif de prélèvement étant respectivement couplés par complémentarité de forme et/ou par adhérence à un raccord de fixation (11, 12) et étant ainsi disposés à une distance longitudinale (x) d'une zone de contact (x15) définie par une partie longitudinale du dispositif de maintien, afin de permettre à un utilisateur d'entrer en contact manuellement avec le dispositif de maintien.

13. Utilisation d'au moins un dispositif de maintien de la technique médicale (10) selon la revendication 3, ou selon l'une des revendications 4 à 9, dans la mesure où elle dépend de la revendication 3, ou selon la revendication 10, dans laquelle la spatule et/ou le dispositif de prélèvement sont respectivement couplés par complémentarité de forme et/ou par adhérence à un raccord de fixation (11, 12) et sont ainsi disposés à une distance longitudinale (x) d'une zone de contact (x15) définie par un segment longitudinal du dispositif de maintien, afin de permettre à un utilisateur d'entrer en contact manuellement avec le dispositif de maintien (10), la caméra (4) étant notamment couplée au moyen d'un mécanisme de serrage ou d'encliquetage, et la caméra (4) comporte un module de communication pour la transmission sans fil de données d'image, les données d'image étant capturées au moyen de la caméra et transmises sans fil au moyen du module de communication, en particulier vers au moins un ordinateur d'un cabinet médical ou d'un hôpital.

14. Procédé de fabrication d'un dispositif de maintien de la technique médicale (10) conçu pour maintenir des dispositifs ou des instruments médicaux généraux, chirurgicaux ou diagnostiques, en particulier des dispositifs ou des instruments de diagnostic pour la cavité buccale et le pharynx, parmi le groupe suivant : spatule, dispositif de prélèvement, source lumineuse, caméra ou autres moyens auxiliaires de diagnostic similaires, à savoir un dispositif de maintien de la technique médicale selon l'une des revendications 1 à 10, le procédé de fabrication comprenant au moins les étapes suivantes :
- le formage, en particulier le formage à chaud d'un tube dans la zone d'au moins une extrémité libre du tube, en particulier de manière à former un rétrécissement ou un aplatissement de l'extrémité libre ;
- la formation d'une butée par un formage supplémentaire de ladite au moins une extrémité libre sur une section longitudinale prédéfinie, en particulier à une épaisseur au moins approximativement uniforme, par exemple dans la plage de 0,5 à 1 cm, et la conception ainsi de la butée pour un verrouillage automatique de la spatule et/ou du dispositif de prélèvement ;
- la formation d'un raccord de fixation du dispositif de maintien de la technique médicale (10) pour le montage réversible par adhérence et par serrage de la caméra, notamment en dotant le tube d'un rétrécissement permettant le montage de la caméra dans une position longitudinale prédéfinie.

15. Procédé selon la revendication précédente, dans lequel, à partir d'un produit semi-fini en tube de cuivre, les étapes suivantes sont réalisées :
1/ rétrécissement ou aplatissement dans la zone d'une extrémité libre du tube à une distance longitudinale de l'extrémité libre ;
2/ rétrécissement ou aplatissement supplémentaire de l'ensemble de l'extrémité libre jusqu'à la position longitudinale préalablement définie ;
3/ insertion d'une spatule ou d'un contour de spatule indéformable/plus indéformable dans l'extrémité libre ;
4/ rétrécissement ou aplatissement supplémentaire de l'ensemble de l'extrémité libre jusqu'au contact de la surface de l'enveloppe intérieure avec la spatule ou le contour de spatule.
